# EUROPEAN PATENT SPECIFICATION

(11) **EP 3 497 072 B1**
(45) Date of publication and mention of the grant of the patent: **19.02.2020**
(21) Application number: 18730096.7
(22) Date of filing: 26.04.2018
(51) Int. Cl.: C07C 1/20, C07C 11/06

(54) **A SYSTEM FOR REACTOR TEMPERATURE CONTROL**
SYSTEM ZUR REAKTORTEMPERATURREGELUNG
SYSTÈME DE RÉGULATION DE TEMPÉRATURE DE RÉACTEUR

(30) Priority: 27.04.2017 IR 13963001286
(43) Date of publication of application: 19.06.2019
(73) Proprietor: National Petrochemical Company (P.J.S), Tehran 1993834557 (IR)
(72) Inventor: MAJEDI ASL, Zohre, Tehran 1435884711 (IR); SAFARI, Davood, Tehran 1435884711 (IR); ALAEI, Seyed Hadi, Tehran 1435884711 (IR); MEHRABI, Ali, Tehran 1435884711 (IR); BAGHMISHEH, Gholamreza, Tehran 1451754439 (IR); SARBAZI, Samira, Tehran 1435884711 (IR)
(74) Representative: Fuchs Patentanwälte Partnerschaft mbB
(86) International application number: PCT/IB2018/000431
(87) International publication number: WO 2018/197942

(56) References cited:
- CN-A- 103 611 477
- US-A1- 2010 234 655

## Description

The propylene via methanol (PVM) process uses methanol as raw material, and uses six main process production units: reaction and regeneration unit, water washing unit, compression and pre-separation unit, hot separation unit and cold separation unit, and propylene refrigeration unit. The final production products are propylene and ethylene, LPG and gasoline. Among them, the reaction and regeneration unit employs an intercooled adiabatic fixed-bed reactor, uses a dedicated zeolite catalyst, and controls the reaction temperature through a disproportionation reaction to produce propylene. The reaction and regeneration unit is the core technology for the production control process of the methanol to propylene plant process. The temperature control of the reactor directly relates to the propylene yield and the service life of the catalyst. The process parameters of the reaction and regeneration stages must be controlled in the process of production.

In the process of propylene via methanol (PVM), pure methanol is converted into dimethyl ether (DME) and water in presence of aγ-Al₂O₃ catalyst in a firstreactor. Then the output stream of the first reactor enters into PVM reactors. In these reactors, passing over an aluminosilicate zeolite, e.g. ZSM-5, catalyst, DME and methanol are transformed to propylene and a wide range of other hydrocarbons.

A previous temperature control system for PVM reactors uses the temperature of gas side feeds for controlling the temperature of the reactor. This system cannot control the reactor in the desired performance range, and also requires using five high pressure heat exchangers. This invention is aimed at eliminating these five heat exchangers as well as using a system for better control of the reactor temperature. In the process of PVM based on this invention, a new method has been introduced to control temperature of the PVM reactor which uses cold liquid side feeds.

This invention is related to science, chemistry, production of organic materials, and better temperature control of PVM reactor, in class of C07C1/20.

According to the present invention, a control system refers to a system is a combination of components that act together to perform a specific goal.

According to the present invention, a reference input is the actual signal input to the control system.

According to the present invention, a controlled variable (output) is the quantity that must be maintained at prescribed value.

According to the present invention, a disturbance signals refers to unwanted inputs which affect the control-system's output, and resulting in an increase of the system error. The control system should partially eliminate the affects of disturbances on the output and system error.

According to the present invention, a cascade control refers to the following: In single-loop control, the controller's set point is set by an operator, and its output drives a final control element. For example: a level controller driving a control valve to keep the level at its set point. In a cascade control arrangement, there are two (or more) controllers of which one controller's output drives the set point of another controller. For example: a level controller driving the set point of a flow controller to keep the level at its set point. The flow controller, in turn, drives a control valve to match the flow with the set point the level controller is requesting.

The controller driving the set point (the level controller in the example) is called the primary, outer, or master controller receiving the set point (flow controller in the example) is called the secondary, inner or slave controller. According to the present invention, the temperature controller TC is the master controller regulating two control loops, wherein one of the control loops regulates the flow of the gas feed into the PVM reactor, and wherein the other control loop regulates the flow of the liquid feed into the PVM reactor. Preferably, in the present invention, there are at least one flow controller acting as slave controller of cascade loop and another flow controller acts only in special situations, more preferably there are exactly two flow controllers, preferably acting as slave controllers.

According to the present invention the terms "gas/liquid separation drum" and "liquid feed separation drum" and "flash vessel" are used interchangeably.

The term "DME condensate inlet stream" refers to the liquid side feed entering the PVM reactor bed.

According to the present invention, damping disturbances relates to the following: When a disturbance imposes in to a control system, the process value exceeds from the specified set point. The control system should change the opening of control valve and approaches the process value in to the specified set point. Process variable overshoots oscillate around the set point and it finally settles out.

According to the present invention, the terms "temperature controller" and "temperature transmitter" are used interchangeably. Moreover, the terms "flow controller" and "flow transmitter" are used interchangeably.

According to the present invention, valve saturation is indicated by the flat portion of the flow vs. current characteristic. Saturation occurs when there is no relative change in flow, even with a change in current. The saturation current of a normally closed valve is the level at which any increase in current does not increase the flow. The saturation current for a normally open valve is the level where the flow decreases to the expected leakage flow rate. Note: The maximum current may or may not be the same value at which saturation occurs. In other words, the saturation flow is typically at or above the maximum current required for most flow control valves.

In the previous process (Iran Pat. No. 72726), temperature control for a six bed PVM reactor was accomplished using gas part of incoming side feed to the beds of the reactor. The product from the first reactor (DME reactor), including water and dimethyl ether and unconverted methanol, was divided into twoparts. The major part of DME reactor enters a gas/liquid separation drum after a couple of cooling steps up to 140 to 160°C, and liquid stream (water and unreacted methanol) is divided from gas hydrocarbon stream (dimethyl ether). The gas hydrocarbon stream is divided into fivebranches, and then, each stream enters a high pressure heat exchanger and their temperature increase up to 250 to 290°C, they are sprayed on 2nd to 6th beds. Liquid stream, after some more cooling cycles, is divided into fivebranches and enters 2nd to 6th beds with the temperature of 60 to 70°C. Actually in this system, adjusting temperature control of the reactor is done based on temperature of gas as well as liquid side feed flows. This system is unable to damp disturbances and unable to control the reactor. On the other hand, this system uses five high pressure heat exchanger which are faced with leakage and repairing problems, but these kinds of problems have been solved in the present invention.

In China, two propylene via methanol production units have been established. According to notes in Patent No. CN203683415, one of the problems in reactor side feeding system for such units is the leakage of high pressure exchanger in side streams of the reactor. Occurring leakage from high pressure exchangers lead to increase in thermal load of heating equipment, not achieving the temperature of reactor beds to optimum reaction temperature for catalyst, reducing the propylene selectivity and increasing the level and temperature of outgoing waste water with respect to designed level. Therefore, Chinese companies in Patents No. CN203683415 and CN103611477, aiming at eliminating side feed exchangers of the reactor, provided a new control system based on adjusting flow rate of gas and liquid side stream to control the temperature of the reactor.

In US 2010/234655 A1, the inventors propose to control the process for the production of propylene via methanol by regulating the flow of the gaseous feed, i. e. by co-feeding a supplementary stream consisting of olefins and inert gases.

According to the present invention, a new method, and/or a new system is provided for controlling the temperature of a propylene via methanol reactor. This controlling system and/or method preferably includes equipment such as heat exchangers, gas/liquid separation drum, control valves, temperature and flow transmitters. In this system control loops control the temperature of intermediate beds of a propylene via methanol reactor wherein preferably the liquid side feed comes from a separation drum.

For example, if the temperature of bed goes up for any reason, the excess heat should be dissipated from the reactor bed because it could eventually lead to deterioration and deactivation of the catalyst material. The catalyst material can be any known catalyst material capable of catalyzing the reaction in the PVM reactor. Known materials include for example aluminosilicate zeolite, like ZSM-5, preferably having the chemical formula NaₙAlₙSi₉₆₋ₙO₁₉₂·16H₂O(0<n<27). In the PVM reactor dimethyl ether is converted into propylene and other hydrocarbons over the catalyst material.

In the PVM process preferably cold liquid (water and dimethyl ether) showers on the bed to control the temperature rise in the reactor. When the temperature of bed goes up, a temperature transmitter sends a signal to the control valve TV located on the cold liquid feed line and open it to send more cold liquid feed, shower on the catalyst bed and cool it down.

Preferably, the level of liquid existing in the separation drum is important. Therefore, the temperature of the stream coming to the separation drum as well as the level of the tank may pref-erablybe controlled. A bypass stream on the utility stream of the exchanger may preferably be used to control the temperature of drum inlet stream and/or a liquid level controller may preferably be applied to control the level of the separation drum. Also, in this system, it is possible and preferred to provide one heat exchanger to adjust the temperatures for all gas streams to intermediate beds of PVM reactor.

Technical problem and objectives of the invention are the following:
The previous control system which controls the temperature of the reactor using the temperature of the gas side feed is unable to damp disturbances. For example, when the main feed flow rate increases or decreases (±5%) the control system of the prior art cannot damp it, which means it cannot keep the reactor in desired temperature range (around 480 °C). Previous control systems use fivehigh pressure heat exchangers in order to heat gas side feed streams of the reactor. When the temperature of the bed goes up, the temperature transmitter send a signal to the control valve located on the steam line of related heat exchanger and closes it to send less steam. This type of temperature controlling is very slow because it takes a lot of time for the system to respond and controls the bed's temperature.

On the other hand, leakage may occur in high pressure exchangers of side feeds and may lead to increase in thermal load of heating equipment, lack in reaching the optimum reaction temperature in the catalyst beds, reduction of propylene selectivity, increasing the amount of waste water compared with desired value and increasing the temperature of effluent waste water compared with desired value. This invention is intended to remove these fiveheat exchangers as well as using a system to better control the temperature of the reactor.

The solution chosen for this problem is using a cold side feed (liquid feed) to control temperature of the reactor, preferably using latent vaporization heat of a cold side feed. Preferably, control loops for the bed's temperature of the reactor are based on liquid stream separated in a flash vessel (also referred to asgas/liquid separation drum). More preferably, a level controller is used to control the liquid level of the flash vessel (gas/liquid separation drum). Preferably, the side feed stream of the PVM reactor before entering the flash vessel is in two-phase form. Therefore, thermal as well as pressure conditions of feed comingto the flash vessel may highly influence the distribution of gas and liquid phase. Preferably, the feed temperature of the two-phase vessel is controlled using a bypass on the utility stream of exchanger. Preferably, in order to make sure about providing liquid cold feed as well as helping the dynamics of thermal control, the residence time in the flash vessel may be increased.

Hence, to solve the above problem and objectives, the present invention proposes a method for controlling the temperature of a propylene via methanol (PVM) reactor in PVM technology by a temperature controller TC adjusting the flow of the gas feed and/or the liquid feed into the PVM reactor depending on the set point of the TC,
wherein the temperature controller TC acts as a master controller controlling two control loops, wherein one of the control loops regulates the flow of the gas feed into the PVM reactor, wherein the other control loop regulates the flow of the liquid feed into the PVM reactor,
wherein the control loop regulating the liquid side feed is the main control loop and wherein the control loop regulating the gas side feed is the auxiliary control loop,
wherein the PVM reactor comprises several reactor beds, wherein the temperature is regulated in at least one reactor bed, wherein the TC receives the temperature of the PVM reactor downstream of a reactor bed, wherein the gas feed and the liquid feed enter the PVM reactor upstream of the reactor bed, and
wherein the liquid side feed comprises water and dimethyl ether.

Moreover, the present invention relates to a system for controlling the temperature of the propylene via methanol reactor in PVM technology capable of performing the above method comprising a temperature controller TC, used as a main controller, and at least two flow controllers FC and FC-105.

In addition, the present invention relates to the use of the above system for controlling the temperature of an adiabatic fixed bed reactor with a cold liquid side stream.

The advantages of the invention are the following:
The new control system and method provide better and more stable control of the temperature of the propylene via methanol production reactor. After implementing loops of thermal control for the new system in a simulation environment, results of dynamic simulation under different scenarios showed that this strategy, in comparison to previous strategy of Research and Technology Co., as well as the strategy prescribed by the Chinese company, has better performance and avoids thermal deviation of beds from desired temperature. The new control system allows an immediate adaptation of the temperature by adapting the flow of a gas side feed and/or of a liquid side feed and does not rely on the increase in temperature of side feed gas streams as in the above outlined prior art. Another advantage of the invention is that the desired temperature in PVM reactors is in the range of 475 to 485 centigrade degrees. Moreover, the new control system is simpler than the old one.

The invention has industrial applicability. In case of using the control system according to this invention, thermal control of propylene via methanol reactor is achieved more conveniently and effectively. Due to removal of high pressure exchangers of the reactor in this system, the problems caused by such exchangers such as a slow response and leakage are solved and consumption of high vapor pressure is reduced effectively which improves economic of propylene via methanol (PVM) plant. The control system of this invention can also be used for all adiabatic multistage fixed bed reactors with a cold liquid stream as a side feed.

In the PVM process, methanol, as the feed, converts into a range of products including paraffin, olefins as well as aromatic hydrocarbons in two steps. In a first reactor (the DME reactor), methanol converts into dimethyl ether in a catalytic reaction, and then, in a second reactor (the PVM reactor), the intermediate product dimethyl ether participates in another catalytic reaction resulting in the production of propylene alongside with other hydrocarbons. Thermal control of the PVM reactor is very important in orderto reach the maximum desired propylene product.

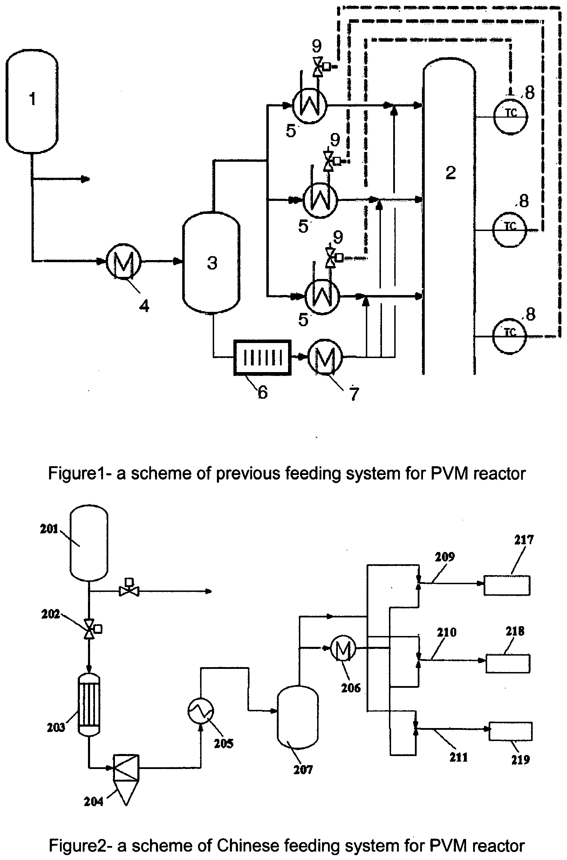

In figure1, a scheme of previous method of Research and Technology Co. to control temperature of PVM reactor is shown. In the previous plan, temperature control of the reactor (2) is accomplished through adjusting the temperature of gas part of side gas streams. In this process, gas stream from top of separation drum (3) is divided into fivebranches, and each branch, after passing through a heat exchanger (5) which works with high pressure vapor, enters second to sixth beds. Measuring the temperature of each bed is done based on the average of reported values by some thermal sensors (8). Controlling the temperature of the bed is done through controlling the level of steam used in a side exchangers (5). Therefore, as an example, if the temperature of a bed exceeds a certain value, the temperature of incoming gas is reduced by reducing the level of high pressure steam in side exchanger related to the gas stream of that bed. The main disadvantage of this control system is the long time it takes before changing the temperature of the gas feed due to a change in the flow rate of steam in the exchangers, and in this time interval, changes in bed temperature may harm the catalyst or reduces the level of producing the desired product. Indeed, due to the low specific heat value of the gas, this system acts slowly and is unable to damp disturbances and control of the system within desired performance range.

In the control system proposed by a Chinese company, the location of the heat exchangers and the method for heating gas streams sent to catalyst bed is like figure 2. This plan uses an exchanger (206) instead of using fiveheat exchangers, and thermal control is achieved through mixingcold and hot gas flows. This plan is also unable to damp disturbances because of using sensible heat of gas stream, and therefore, it is not effective.

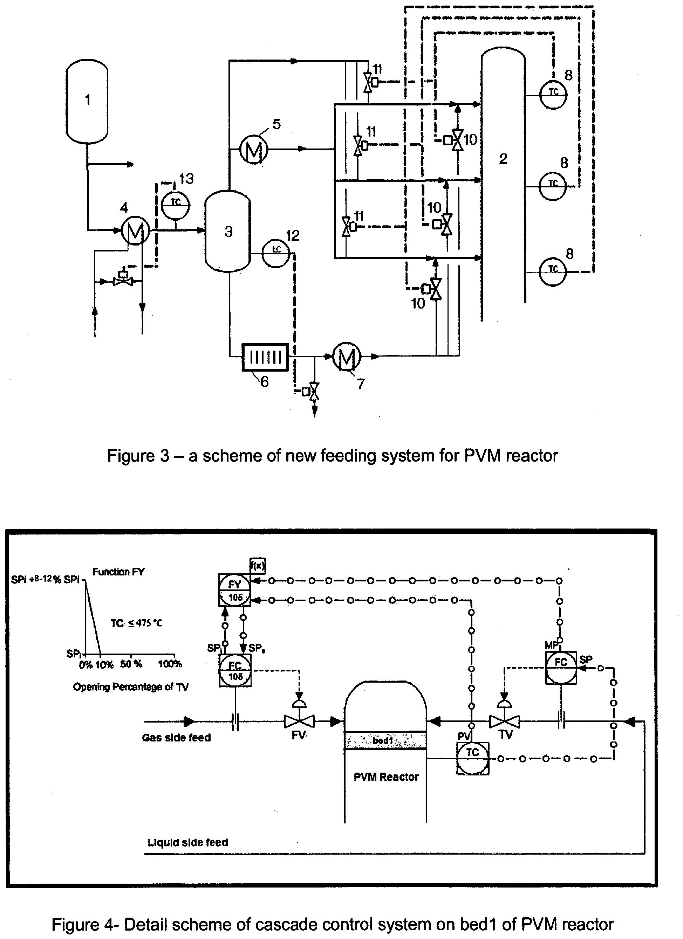

In the method and control system of the present invention, replacing the gaseous temperature controlling feedstream of the prior art with a higher heat capacity fluid has been considered. Figure 3 depicts a scheme of the feeding system according to this invention. In this new method and new system, the temperature of the catalyst beds is controlled by adjusting the flow rate of liquid streams. This act is preferably done using cascade formation of a temperature controller controlling two flow rate controllers, one of which preferably controls a cold liquid side stream (10).

The present invention provides a method for controlling the temperature of a propylene via methanol (PVM) reactor in PVM technology by a temperature controller TC adjusting the flow of the gas feed and/or the liquid feed into the PVM reactor depending on the set point of the TC. This control arrangement preferably reflects a cascade control arrangement. According to the invention, the temperature controller TC acts as a master controller controlling two control loops, wherein one of the control loops regulates the flow of the gas feed into the PVM reactor, and wherein the other control loop regulates the flow of the liquid feed into the PVM reactor. The control loop regulating the liquid side feed is the main control loop and the control loop regulating the gas side feed is the auxiliary control loop.

Generally, in cascade control arrangement, there are at least two controllers of which one controller's output drives the set point of a downstream controller. According to the present invention, a temperature controller TC, is preferably driving the set point of a flow controller FC to keep the temperature of a bed of the PVM reactor at its set point. Preferably, the flow controller FC, in turn, drives a control valve to match the flow with the set point the temperature controller is requesting. Preferably, the flow controller FC is a proportional-integral-derivative (PID) controller. According to the present invention, the second flow controller (FC-105) receive the output of first flow controller (MP) and the temperature value of TC controller. According to these values and the initial set point of itself (SPi, adjusted by the operator), FY-105 calculate the new set point and send it to FC-105.

According to the invention, the PVM reactor comprises several reactor beds, wherein the temperature is regulated in at least one reactor bed and wherein the TC receives the temperature of the PVM reactor downstream of a reactor bed and wherein the gas feed and the liquid feed enter the PVM reactor upstream of the reactor bed.

More preferably, the method and system enables the control of the temperature in at least two reactor beds separately, even more preferably in at least three reactor beds, most preferably, the temperature is controlled separately in each of the reactors bed. When several reactor beds are controlled separately, the flow of the gas feed and/or the liquid feed into each of the PVM reactor beds is controlled using an independent temperature controller for each bed. This means for example that controlling the temperature in two reactor beds involves the use of two temperature controllers TC working independent of each other, wherein each of the temperature controllers controls two control loops as explained above; controlling the temperature in three reactor beds involves the use of three temperature controllers TC working independent of each other each and each controlling two control loops as explained above.

The set point of the temperature controller TC is preferably set to a specified range, preferably to a range of 475°C to 485°C. More preferably, the set point set at the temperature controller is set to a value of 480 °C +/- 5°C. Even more preferably, the set point at the temperature controller TC is set to a value of 480 °C.

In case the temperature received from the PVM reactor exceeds the set pointset or set point range set at the TC, preferably a step of adjusting the flow of the liquid feed into the PVM reactor is performed using a flow controller FC located in the main control loop, more preferably by increasing the flow of the liquid feed, even more preferably by increasing the opening of the valve TV located on the liquid side feed line.

In case the temperature received from the PVM reactor is below or at the set pointof the temperature controller TC, preferably a step of adjusting the flow of the gas feed into the PVM reactor is performed, using a flow controller FC-105 located in the auxiliary control loop.

Figure 4 reveals details of the cascade control system. Preferably, the temperature of each bed of the PVM reactor is controlled by using two control loops; preferably one main control loop and one auxiliary control loop. Preferably, the main control loop comprises a cascade controller that adjusts the temperature to a set point set at the temperature controller TC by changing the liquid side feed entering to the bed; preferably, the liquid side feed comprises dimethyl ether (DME) condensate and water. When the temperature rises above the set point that is set at the temperature controller TC, preferably the cascade controller increases the opening percentage of the control valve TV installed on the DME condensate inlet stream to the bed, so the temperature drops to the set pointthat is set at the temperature controller TC.

Preferably, if the temperature of the bed drops below said set point, while the temperature is greater than 475°C, the temperature is still controlled by the cascade controller. When the temperature reaches values lower than 475°C, the auxiliary control loop adjusts the gas side feed, preferably, through increasing the gas side feed.Preferably, the gas side feed comprises DME. According to the present invention, the gas side feed represents a hot side feed increasing the temperature in the PVM reactor and/or PVM reactor bed, and the liquid side feed represents a cold side feed decreasing the temperature in the PVM reactor and/or PVM reactor bed.

Preferably, the step of adjusting the gas feed involves a function unit FY-105 is in connection with flow controller FC-105. More preferably, the function unit FY-105 receives a manipulated value MP of the flow controller FC corresponding to the degree of opening of the valve TV located on the liquid feed line and the temperature process value from TC

Even more preferably,the control unit FY-105 calculates an external set point SPe depending on the manipulated value MP, this external set point is sent to the flow controller FC-105 for regulating the flow at the gas flow controller FC-105. Preferably, SPe corresponds to SPi + 3 % to 20 % SPi, preferably SPi + 5 to 12% SPi, wherein SPi is an internal set point set by the operator at the flow controller FC-105.

For this purpose, the following function may preferably be implemented: Preferably, when the temperature of the bed is below 475°C and the manipulated value of the cascade controller, corresponding to an opening percentage of the valve TV is greater than 3 % to 20 %, preferably greater than 5 % to12% the output of the control unit FY-105 is equal to an initial internal set point of FC-105 adjusted by an operator (internal set point). When the manipulated value of the cascade controller corresponding to the opening percentage of the valve TV is lower than 3 % to 20 %, preferably lower than 5 % to 12%, the output of the control unit FY is increasing from the initial set point SPi to an external set point SPe =SPi+(5 % to 12)%SPi in line with the opening percentage of the control valve installed on the liquid feed stream to the bed. Preferably, the cascade controller is the flow controller FC. This output of FY-105 is preferably sent as a remote (external) set point SPe to the controller FC-105. Preferably, then the gas flow increases, resulting in temperature rising to meet the set point at the temperature controller TC.

As figure 3 shows, the temperature of the gas stream is preferably also controlled and adjusted by a heat exchanger(5). Even more preferably, the temperature of the gas feed is adjusted upstream of the flow controller FC-105 using an additional heat exchanger (5) and also the temperature of the liquid feed is adjusted upstream of the flow controller FC by using an additional heat exchanger (6, 7).

In order to implement the desired strategy, in addition to developing a cascade strategy upon temperature controllers, preferably an outgoing stream was added to a gas/liquid phase separator drum (3) to control the level of the liquid on it. The stream coming to the gas/liquid phase separator drum (3) is in two-phase form, therefore, a little change in its temperature causes change in the quality of the stream, and a change in the flow rate of the liquid as well as the gas streams coming out of the separator. Under these circumstances, a high load is applied to the process. In previous plan of Research and Technology Co., the temperature of this stream was controlled manually, and in order to avoid changes in the quality of the incoming stream and its consequences, temperature controller (13) is preferably used in the new design.

Preferably, in the new designed controlling system, the level controller of the liquid phase in the two-phase separator (3) may be very important. To achieve controlling the liquid level, an outgoing stream may preferably be used. To stop increasing the level of liquid in the flash vessel, it may preferably bepossible to return this liquid stream to the system by using the major control loop. In apreferred method, in order to stop the overflow of the vessel, normal performance of the liquid control valves TV change, and first priority will be controlling the level of separator using a level controller (12). Preferably, this extra stream works for switch high, and separate exceeded liquids from the vessel, and will not be in service in normal conditions of the unit. More preferably, using this strategy allows proper control of the temperature of intermediate beds.

The system for controlling the temperature of the propylene via methanol reactor in PVM technology according to the present invention comprises at least a temperature controller TC, used as a main controller, and at least two flow controllers (FC and FC-105).

Preferably, the system according to the present invention comprises per PVM reactor bed: one temperature controller TC and two flow controllers, FC and FC-105, that are located downstream of the temperature controller TC. Preferably, these flow controllers are slave controllers of the temperature controller TC. Preferably, the flow controller FC controls the liquid side feed in the main control loop and the flow controller FC-105 controls the gas side feed in the auxiliary side feed.lt is preferred that downstream of the flow controller FC there is a valve TV for controlling the flow of the liquid side feed to the PVM reactor and downstream of the flow controller FC-105 there is a valve FV for controlling the gas side feed to the PVM reactor.

Preferably, the system comprises the additional control unit FY-105 located downstream of the temperature controller but upstream of the flow controller FC-105. The control unit FY-105 preferably receives a signal of the temperature controller TC and an additional signal of the flow controller FC and calculates an external set point according to these signals. Preferably, the additional signal received from the flow controller FC is the manipulated value corresponding to the opening percentage of the valve TV.

Preferably, the system comprises a gas/liquid separation drum located upstream of the liquid side feed line and the gas side feed line. Preferably, in the gas/liquid separation drum dimethyl ether product received in two-phase form from the DME reactor is separated. Preferably, this phase separation drum comprises an upstream temperature controller and/or a downstream level controller.

### Examples

In the following, a scenario is considered wherein the detected temperature of the bed is 465°C for any process problem reason. The cascade controller according to the present invention will decrease the opening of the valve TV and less liquid stream is sent to the reactor bed and then the temperature will rise. The inventors designed the control system in a way that when the temperature is below 475°C and the manipulated value (TV valve opening) of the cascade controller is greater than (5 to 12)% nothing happens and the output of FY-105 is equal to the initial set point of FC-105 adjusted by operator (internal set point of gas feed flow). But if the temperature still does not reach to the adjusted set point, and the opening of TV valve (MP) is less than (5-12) %, then MP value and the process value (bed's temperature) are sent to FY-105. The output of FY-105 is increasing from SPi to SPi+(5 to 12)%SPi according to the opening percentage of the control valve installed on the liquid feed stream to the bed. This output is sent as remote (external) set point to the FC-105, then the gas flow increases, resulting in temperature rising to meet the set point at the temperature controller TC.

In order to examine the performance of new controlling system, first, steady simulation of the reactor and feeding system has been implemented, and after validation, equipment has been designed. After implementing control loops, dynamic simulation has been achieved, and then by applying disturbances in streams and possible conditions in this system, temperature control has been evaluated. In order to evaluate the performance of controlling system, a couple of scenarios have been defined, and by observing and drawing the reply from the system, performance evaluation takes place. Defined scenarios are as follows:
**Example1:** 5 percent of increase and 10 percent of decrease in gas feed flow rate coming from the top of catalyst reactor: The flow rate of feed streams to process is one of incoming specifications that are possible to change. Due to increase in the flow rate, the level of heat produced in the bed increases and the temperature of the bed increases as well. In this condition, it is necessary for the controller to act and stop increasing the temperature of the bed. Reduction in feed flow rate will have the reverse effect.
**Example2:** 5 percent of increase and 10 percent of decrease in liquid feed: flow rate of feed streams to process are among incoming specifications that are possible to be changed. By increasing the flow rate of the liquid, first, the level of produced liquid in phase separator increases, and if the outgoing flow rate is not adjusted well, the height of the liquid will increase. In other side, increase of the liquid feed flow rate leads to increase in the feed flow to the reactor which increases the temperature of the bed. This thermal increase has to be adjusted with thermal control loops. Reduction in incoming liquid leads to reduction in flow rate of the height of the vessel, and in order to stop emptying of 2-phase separator, the flow rate of outgoing liquid has to be adjusted by controller. The effect of this flow rate decrease over the temperature of the reactor has to be removed by temperature control loops as well.
**Example3:** 5 percent increase and 10 percent of decrease in conversion rate of main reaction for catalytic reactors: in real situation, conversion rate of the reaction is a function of temperature, pressure and the combination percentage, meanwhile, due to lack of access to synthetic of the reaction, the level of conversion rate has been assumed to be constant in this simulation. In order to evaluate the performance of controller in presence of model error, the conversion rate first increases by 5 percent, and then decreases by 10 percent. Increasing the conversion rate through increasing the heat of the reaction leads to increase the temperature of the bed, and reversely, its reduction causes the reduction in the temperature of the bed. In this example, conversion rate for the following reactions are changed:

   1) *C*₃*H*₆ + 3*H*₂*O* → 3*CH*₃*OH*

   2) *CH*₃*OCH*₃ +*H*₂ → 2*CH*₃*OH*

   3) 1000*CH*₃*OH* → 999*H*₂*O* + 118*C*₃*H*₆ +...
**Example4:** 5 percent of increase and 10 percent of decrease in the temperature of liquid feed: the temperature of the feed is one of specifications and might change any times. Increasing or decreasing the liquid feed flow leads to change in the temperature of the beds of reactor which is required to be adjusted by temperature control loops.
**Example5:** increase or decrease of the temperature in gas feed for +5, and then -10: The temperature of gas feed is one of specifications that may change any times. Increase or decrease in this feed leads to increase or decrease in the temperature of reactor beds which is required to be changed by temperature control loops.

In previous strategy, there is only 1 case out of 5 above mentioned examples in which it was possible to control the system and damp disturbances. In this controlling method, adjusting vapor temperature (at the range of 150°C to 200°C) is used which has a very low heat capacity. On the other hand, heat load applied to system due to each disturbance is so much higher. As an example, in example2, there is the increase or decrease of energy for 100MJ. Meanwhile, controlling system is only able to damp about 0.5MJ of this disturbance.

The strategy of Chinese company was only able to control system and damp the disturbance in 2 cases out 5 above mentioned examples. These two cases are 1st and 3rd examples. This controlling strategy, like the previous one, used adjusting vapor temperature (range of 150°C to 200°C) as controlling variable, and due to low heat capacity of the gas, the system is unable to control and damp the disturbance. Indeed, because of using the gas with constant flow rate (with different temperatures) and using sensible heat, control with proper performance was not practically observed in case of disturbances.

In this invention, liquid streams have the ability of higher heat absorption due to higher latent vaporization heat, and therefore, in comparison to the strategy based on gas stream flow rate, they showed better performance under defined scenarios and avoided deviation of the beds temperature from desired value. This strategy was able to perform well in 4 out of 5 examples, and in 4th example (change in the temperature of the feed at the top of reactor), the temperature of all beds were controlled except two of them. In comparison between this strategy as well as Chinese strategy, the speed of the control based on liquid flow rate was so much higher than Chinese counterpart under 1st and 3rd example.

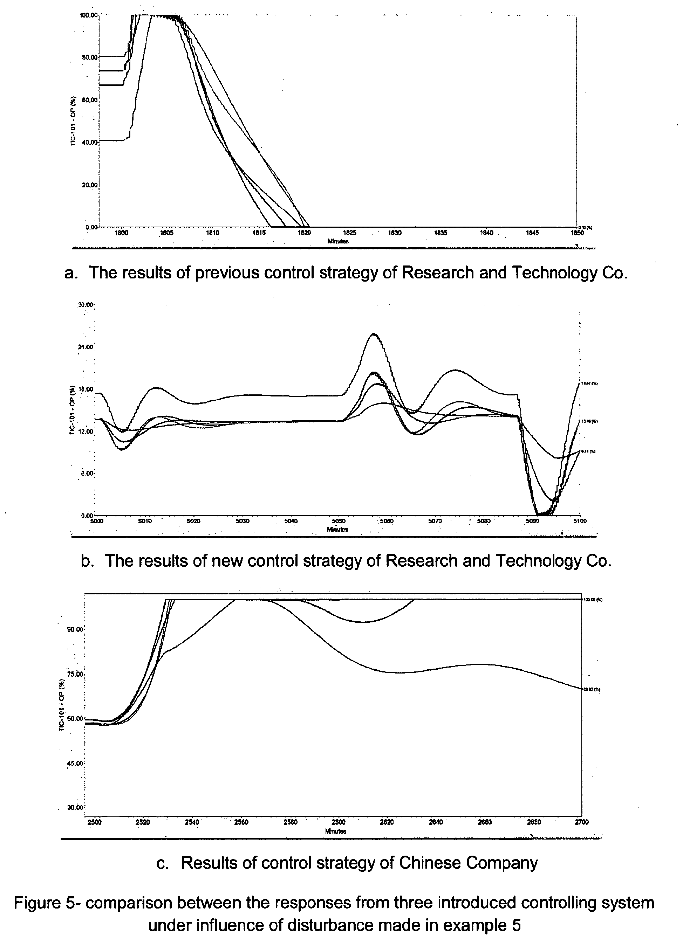

Figure 5 shows an example of comparing the reaction of three introduced control systems under influence of disturbance made in example5. Previous strategy of the company and Chinese strategy valves are saturated, but the new strategy for this invention is able to damp this disturbance. But, in the case of emptying of the two-phase separation vessel (3), the temperature of the beds is out of control which is controllable using the controller of the level that is claimed in this invention.

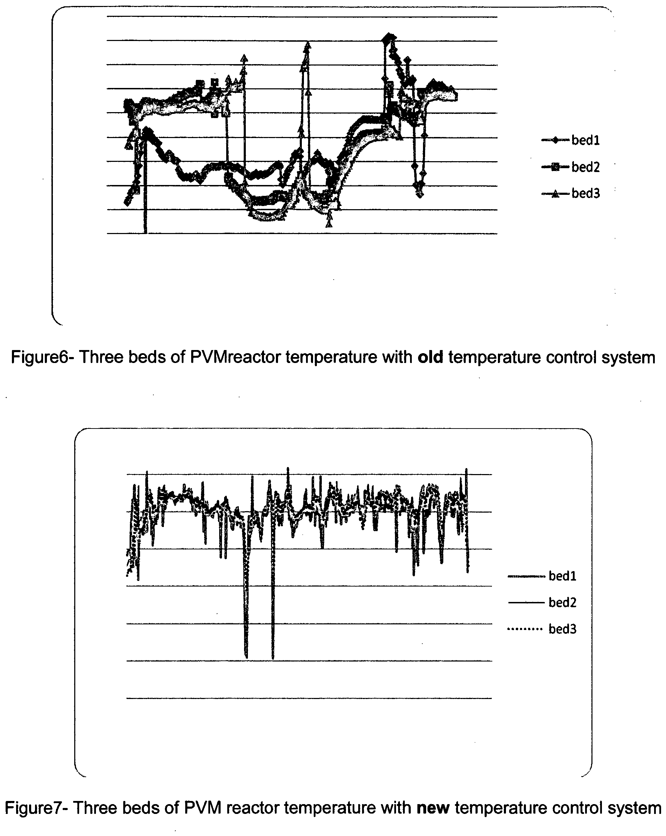

New control system is used in a demonstration plant with a production of 1 ton/day of propylene. The results asshownin figures 6 and 7 reflect the temperature regulation before and after changes in three beds of the PVM reactor.

### Description of the figures:

Figure1 represents a scheme of previous feeding system for PVM reactor.
Figure2 represents a scheme of Chinese feeding system for PVM reactor.
Figure 3 represents a scheme of new feeding system for PVM reactor.
Figure 4 represents a detailed scheme of the cascade control system on bed1 of PVM reactor.
   a. The results of previous control strategy of Research and Technology Co.
   b. The results of new control strategy of Research and Technology Co.
   c. Results of control strategy of Chinese company.
Figure 5 represents a comparison between the responses from three introduced controlling system under influence of disturbance made in example 5.
Figure 6 depicts a temperature curve of three beds of a PVM reactor with the old temperature control system.
Figure 7 depicts a temperature curve of three beds of a PVM reactor temperature with the new temperature control system according to the present invention.

## Claims

1. A method for controlling the temperature of a propylene via methanol (PVM) reactor in PVM technology by a temperature controller TC adjusting the flow of the gas feed and/or the liquid feed into the PVM reactor depending on the set point of the TC,
wherein the temperature controller TC acts as a master controller controlling two control loops, wherein one of the control loops regulates the flow of the gas feed into the PVM reactor, wherein the other control loop regulates the flow of the liquid feed into the PVM reactor,
wherein the control loop regulating the liquid side feed is the main control loop and wherein the control loop regulating the gas side feed is the auxiliary control loop,
wherein the PVM reactor comprises several reactor beds, wherein the temperature is regulated in at least one reactor bed, wherein the TC receives the temperature of the PVM reactor downstream of a reactor bed, wherein the gas feed and the liquid feed enter the PVM reactor upstream of the reactor bed, and
wherein the liquid side feed comprises water and dimethyl ether.

2. Method according to claim 1, wherein the set point at the TC is set to a range of 470=°C to 485°C.

3. Method according to any of the preceding claims, wherein in case the temperature received from the PVM reactor is above the set point of the TC, a step of adjusting the flow of the liquid feed into the PVM reactor is performed using a flow controller FC located in the main control loop, preferably by increasing the flow of the liquid feed, more preferably by increasing the opening of the valve TV located on the liquid side feed line.

4. Method according to any of the preceding claims, wherein in case the temperature received from the PVM reactor is below or at the set point of the TC, a step of adjusting the flow of the gas feed into the PVM reactor is performed, using a flow controller FC-105 located in the auxiliary control loop.

5. Method according to any of the preceding claims, wherein the step of adjusting the gas feed involves a function unit FY-105 is in connection with flow controller FC-105-.

6. Method according to claim 5, wherein the function unit FY-105 receives a manipulated value MP of the flow controller FC corresponding to the degree of opening of the valve TV located on the liquid feed line.

7. Method according to claim 6, wherein the function unit FY-105 calculates an external set point SPe depending on the manipulated value MP regulating the flow controller FC-105 and the temperature value of TC

8. Method according to claim 7, wherein SPe corresponds to SPi + 3 % to 20 % SPi, preferably SPi + 5 to 12% SPi, wherein SPi is an internal set point set by the operator at the flow controller FC-105.

9. Method according to claims 7 or 8, comprising adjusting the gas liquid feed to the external set point SPe in case the manipulated value MP corresponds to less than 20 %, preferably to a range between 5% and 12 % of the opening of the valve TV.

10. Method according to any of claims 4 to 9, wherein the temperature of the gas feed is adjusted upstream of the flow controller FC-105 and also wherein the temperature of the liquid feed is adjusted upstream of the flow controller FC.

11. System for controlling the temperature of the propylene via methanol reactor in PVM technology capable of performing the method according to claims 1 to 10 comprising:
a temperature controller TC, used as a main controller, and at least two flow controllers FC and FC-105.

12. System according to claim 11, additionally comprising a function unit FY-105.

13. Use of the system according to claim 11 or 12 for controlling the temperature of an adiabatic= fixed bed reactor with a cold liquid side stream.

## Patentansprüche

1. Verfahren zur Regelung der Temperatur eines Methanol-zu-Propylen (PVM)-Reaktors in PVM-Technologie durch eine Temperaturregelung TC, die den Strom der Gaszufuhr und/oder der Flüssigkeitszufuhr in den PVM-Reaktor abhängig vom Sollwert der TC anpasst,
wobei die Temperaturregelung TC als eine Hauptregelung, die zwei Regelkreise regelt, arbeitet, wobei einer der Regelkreise den Strom der Gaszufuhr in den PVM-Reaktor steuert, wobei der andere Regelkreis den Strom der Flüssigkeitszufuhr in den PVM-Reaktor steuert,
wobei der Regelkreis, der die Flüssigkeitseite-Zufuhr steuert, der Hauptregelkreis ist und wobei der Regelkreis, der die Gasseite-Zufuhr steuert, der Hilfsregelkreis ist, wobei der PVM-Reaktor mehrere Reaktorbetten umfasst, wobei die Temperatur in mindestens einem Reaktorbett gesteuert wird, wobei die TC die Temperatur des PVM-Reaktors nachgelagert bezüglich eines Reaktorbetts empfängt, wobei die Gaszufuhr und die Flüssigkeitszufuhr vorgelagert bezüglich dem Reaktorbett in den PVM-Reaktor eintreten, und
wobei die Flüssigkeitseite-Zufuhr Wasser und Dimethylether umfasst.

2. Verfahren gemäß Anspruch 1, wobei der Sollwert an der TC auf einen Bereich von 470°C bis 485°C eingestellt wird.

3. Verfahren gemäß einem der vorhergehenden Ansprüche, wobei im Falle, dass die Temperatur, die von dem PVM-Reaktor empfangen wird, über dem Sollwert der TC liegt, ein Schritt von Anpassen des Stroms der Flüssigkeitszufuhr in den PVM-Reaktor unter Verwendung einer Durchflussregelung FC, die im Hauptregelkreis lokalisiert ist, durchgeführt wird, bevorzugt durch Erhöhen des Stroms der Flüssigkeitszufuhr, stärker bevorzugt durch Vergrößern der Öffnung des Ventils TV, das an der Flüssigkeitseite-Zufuhrleitung lokalisiert ist.

4. Verfahren gemäß einem der vorhergehenden Ansprüche, wobei im Falle, dass die Temperatur, die von dem PVM-Reaktor empfangen wird, unter dem oder beim Sollwert der TC liegt, ein Schritt von Anpassen des Stroms der Gaszufuhr in den PVM-Reaktor unter Verwendung einer Durchflussregelung FC-105, die im Hilfsregelkreis lokalisiert ist, durchgeführt wird.

5. Verfahren gemäß einem der vorhergehenden Ansprüche, wobei der Schritt von Anpassen der Gaszufuhr eine Funktionseinheit FY-105 in Verbindung mit Durchflussregelung FC-105 einbezieht.

6. Verfahren gemäß Anspruch 5, wobei die Funktionseinheit FY-105 einen manipulierten Wert MP von der Durchflussregelung FC entsprechend dem Öffnungsgrad des Ventils TV, das an der Flüssigkeitszufuhrleitung lokalisiert ist, empfängt.

7. Verfahren gemäß Anspruch 6, wobei die Funktionseinheit FY-105 einen externen Sollwert SPe abhängig von dem manipulierten Wert MP, der die Durchflussregelung FC-105 steuert, und dem Temperaturwert von TC berechnet.

8. Verfahren gemäß Anspruch 7, wobei SPe SPi + 3 % bis 20 % SPi, bevorzugt SPi + 5 bis 12 % SPi entspricht, wobei SPi ein interner Sollwert ist, der durch den Bediener an der Durchflussregelung FC-105 eingegeben wird.

9. Verfahren gemäß den Ansprüchen 7 oder 8, umfassend Anpassen der Gas/Flüssigkeit-Zufuhr an den externen Sollwert SPe im Falle, dass der manipulierte Wert MP weniger als 20 %, bevorzugt einem Bereich zwischen 5 % und 12 % der Öffnung des Ventils TV entspricht.

10. Verfahren gemäß einem der Ansprüche 4 bis 9, wobei die Temperatur der Gaszufuhr vorgelagert bezüglich der Durchflussregelung FC-105 angepasst wird und wobei auch die Temperatur der Flüssigkeitszufuhr vorgelagert bezüglich der Durchflussregelung FC angepasst wird.

11. System zum Regeln der Temperatur des Methanol-zu-Propylen-Reaktors in PVM-Technologie, das zum Durchführen des Verfahrens gemäß den Ansprüchen 1 bis 10 in der Lage ist, umfassend:
eine Temperaturregelung TC, die als eine Hauptregelung verwendet wird, und mindestens zwei Durchflussregelungen FC und FC-105.

12. System gemäß Anspruch 11, zusätzlich umfassend eine Funktionseinheit FY-105.

13. Verwendung des Systems gemäß Anspruch 11 oder 12 zum Regeln der Temperatur eines adiabatischen Festbettreaktors mit einem kalten/flüssigen Nebenstrom.

## Revendications

1. Procédé pour réguler la température d'un réacteur propylène via méthanol (PVM) en technologie PVM par un régulateur de température TC réglant l'écoulement de la charge d'alimentation gazeuse et/ou de la charge d'alimentation liquide dans le réacteur PVM en fonction du point de consigne du TC,
dans lequel le régulateur de température TC agit en tant que régulateur principal commandant deux boucles de régulation, dans lequel une des boucles de régulation régule l'écoulement de la charge d'alimentation gazeuse dans le réacteur PVM, dans lequel l'autre boucle de régulation régule l'écoulement de la charge d'alimentation liquide dans le réacteur PVM,
dans lequel la boucle de régulation régulant la charge d'alimentation côté liquide est la boucle de régulation principale et dans lequel la boucle de régulation régulant la charge d'alimentation côté gaz est la boucle de régulation auxiliaire,
dans lequel le réacteur PVM comprend plusieurs lits de réacteur, dans lequel la température est régulée dans au moins un lit de réacteur, dans lequel le TC reçoit la température du réacteur PVM en aval d'un lit de réacteur, dans lequel la charge d'alimentation gazeuse et la charge d'alimentation liquide pénètrent dans le réacteur PVM en amont du lit de réacteur, et
dans lequel la charge d'alimentation côté liquide comprend de l'eau et de l'éther de diméthyle.

2. Procédé selon la revendication 1, dans lequel le point de consigne au niveau du TC est fixé à une plage de 470 °C à 485 °C.

3. Procédé selon l'une quelconque des revendications précédentes, dans lequel dans le cas où la température reçue du réacteur PVM est supérieure au point de consigne du TC, une étape de réglage de l'écoulement de la charge d'alimentation liquide dans le réacteur PVM est effectuée au moyen d'un régulateur de débit FC situé dans la boucle de régulation principale, de préférence en augmentant le débit de la charge d'alimentation liquide, mieux encore en augmentant l'ouverture de la soupape TV située sur la conduite d'alimentation côté liquide.

4. Procédé selon l'une quelconque des revendications précédentes, dans lequel dans le cas où la température reçue du réacteur PVM est inférieure ou égale au point de consigne du TC, une étape de réglage de l'écoulement de la charge d'alimentation gazeuse dans le réacteur PVM est effectuée, au moyen d'un régulateur de débit FC-105 situé dans la boucle de régulation auxiliaire.

5. Procédé selon l'une quelconque des revendications précédentes, dans lequel l'étape de réglage de la charge d'alimentation gazeuse fait intervenir une unité fonctionnelle FY-105 en liaison avec le régulateur de débit FC-105.

6. Procédé selon la revendication 5, dans lequel l'unité fonctionnelle FY-105 reçoit une valeur manipulée MP du régulateur de débit FC correspondant au degré d'ouverture de la soupape TV située sur la conduite d'alimentation en liquide.

7. Procédé selon la revendication 6, dans lequel l'unité fonctionnelle FY-105 calcule un point de consigne externe SPe en fonction de la valeur manipulée MP régulant le régulateur de débit FC-105 et de la valeur de température de TC.

8. Procédé selon la revendication 7, dans lequel SPe correspond à SPI + 3 % à 20 % de Spi, de préférence SPI + 5 à 12 % de Spi, dans lequel Spi est un point de consigne interne fixé par l'exploitant au niveau du régulateur de débit FC-105.

9. Procédé selon les revendications 7 ou 8, comprenant le réglage de la charge d'alimentation gazeuse liquide sur le point de consigne externe SPe dans le cas où la valeur manipulée MP correspond à moins de 20 %, de préférence à une plage entre 5 % et 12 % de l'ouverture de la soupape TV.

10. Procédé selon l'une quelconque des revendications 4 à 9, dans lequel la température de la charge d'alimentation gazeuse est réglée en amont du régulateur de débit FC-105 et également dans lequel la température de la charge d'alimentation liquide est réglée en amont du régulateur de débit FC.

11. Système pour réguler la température du réacteur propylène via méthanol en technologie PVM, capable d'exécuter le procédé selon les revendications 1 à 10 et comprenant :
un régulateur de température TC, utilisé comme régulateur principal, et au moins deux régulateurs de débit FC et FC-105.

12. Système selon la revendication 11, comprenant en outre une unité fonctionnelle FY-105.

13. Utilisation du système selon les revendications 11 ou 12 pour réguler la température d'un réacteur à lit fixe adiabatique avec un courant froid côté liquide.
